# EUROPEAN PATENT APPLICATION

(11) **EP 4 052 633 A1**
(43) Date of publication of application: **07.09.2022**
(21) Application number: 20881956.5
(22) Date of filing: 27.10.2020
(51) Int. Cl.: A61B 1/267

(54) **IMAGE CAPTURING DEVICE AND IMAGE CAPTURING SYSTEM**

(30) Priority: 31.10.2019 JP 2019198736
(71) Applicant: Aillis Inc., Chiyoda-ku Tokyo 100-0006 (JP)
(72) Inventor: YASUMI, Takashi, Tokyo 100-0006 (JP); SAKUMA, Akiho, Tokyo 100-0006 (JP)
(74) Representative: Reeve, Nicholas Edward
(86) International application number: PCT/JP2020/040329
(87) International publication number: WO 2021/085443

(57) **Abstract**

Provided are an image capturing device and an image capturing system, which are suitable to make a diagnosis based on an object image obtained by capturing an intraoral image. Provided is an image capturing apparatus, including: a main body which has a proximal end and a distal end, and is formed into a columnar shape having a predetermined length between the proximal end and the distal end so as to enable insertion of at least the distal end into an oral cavity; one or a plurality of light sources which are provided on the proximal end side or the distal end side of the main body, or to the main body, and are configured to emit light having a predetermined frequency band; a diffuser plate which is provided on the proximal end side or the distal end side of the main body, or to the main body, and is configured to diffuse the light emitted from the light sources toward the oral cavity; and a camera which is provided on the proximal end side or the distal end side of the main body, or to the main body, and is configured to capture an object image based on reflected light which has been diffused by the diffuser plate and reflected from the oral cavity.

## Description

### Technical Field

This disclosure relates to an imaging apparatus and an imaging system for imaging an intraoral image of a subject person.

### Background Art

Hitherto, there has been known a diagnosis of, for example, a viral cold based on a doctor's observation of a change in oral state of a subject person. Non-Patent Literature 1 reports that lymph follicles appearing at a deepest part of a pharynx in an oral cavity have a pattern peculiar to influenza. Lymph follicles having the peculiar pattern are referred to as "influenza follicles". Influenza follicles are a characteristic sign of influenza, and are considered to appear about two hours after the onset of influenza. However, appropriate identification of influenza follicles requires intensive training through a large number of cases, and thus is not easy for general doctors.

### Citation List

### Non Patent Literature

[NPL 1] "Posterior Pharyngeal Wall Follicles as a Diagnostic Marker of Influenza During Physical Examination: Considering Their Meaning and Value" by Miyamoto and Watanabe, Journal of Nihon University Medical Association 72(1): 11 to 18 (2013).

### Summary of Invention

### Technical Problem

Thus, in view of the above-mentioned technology, this disclosure has an object to provide an imaging apparatus and an imaging system according to various embodiments, which are suitable to make a diagnosis based on an object image obtained by imaging an intraoral image.

### Solution to Problem

According to one aspect of this disclosure, there is provided "an imaging apparatus, including: a main body which has a proximal end and a distal end, and is formed into a columnar shape having a predetermined length between the proximal end and the distal end so as to enable insertion of at least the distal end into an oral cavity; one or a plurality of light sources which are provided on the proximal end side or the distal end side of the main body, or to the main body, and are configured to emit light having a predetermined frequency band; a diffuser plate which is provided on the proximal end side or the distal end side of the main body, or to the main body, and is configured to diffuse the light emitted from the light sources toward the oral cavity; and a camera which is provided on the proximal end side or the distal end side of the main body, or to the main body, and is configured to image an object image based on reflected light which has been diffused by the diffuser plate and reflected from the oral cavity."

According to one aspect of this disclosure, there is provided "an imaging system, including: the above-mentioned imaging apparatus; and a processing device which is connected to the above-mentioned imaging apparatus in a wired or wireless manner so as to be communicable with the imaging apparatus, and is configured to process an object image imaged by the above-mentioned imaging apparatus."

### Advantageous Effects of Invention

According to various embodiments of this disclosure, the imaging apparatus and the imaging system, which are suitable to make a diagnosis based on the object image obtained by imaging the intraoral image, can be provided.

The above-mentioned effects are mere exemplifications for convenience of description, and are not limitative. In addition to or in place of the above-mentioned effects, any effects described in this disclosure and effects apparent to those skilled in the art can be obtained.

### Brief Description of Drawings

FIG. 1 is a view for illustrating a state in which an imaging system (1) according to one embodiment of this disclosure is in use.
FIG. 2 is a schematic view of the imaging system (1) according to the embodiment of this disclosure.
FIG. 3 is a block diagram for illustrating a configuration of the imaging system (1) according to the embodiment of this disclosure.
FIG. 4 is a flowchart for illustrating processing executed by a processing device (100) in the embodiment of this disclosure.
FIG. 5 is a flowchart for illustrating processing executed by the processing device (100) in the embodiment of this disclosure.
FIG. 6 is a perspective view for illustrating a configuration of an imaging apparatus (200) according to the embodiment of this disclosure.
FIG. 7A is a schematic view for illustrating a top configuration of the imaging apparatus (200) according to the embodiment of this disclosure.
FIG. 7B is a schematic view for illustrating a side configuration of the imaging apparatus (200) according to the embodiment of this disclosure.
FIG. 8 is a schematic view for illustrating a front configuration of the imaging apparatus (200) according to the embodiment of this disclosure.
FIG. 9 is a perspective view for schematically illustrating a configuration of an auxiliary member (300) in the embodiment of this disclosure.
FIG. 10 is a side view for schematically illustrating a side configuration of the auxiliary member (300) in the embodiment of this disclosure.
FIG. 11 is a schematic view for illustrating a state in which the imaging apparatus (200) and the auxiliary member (300) in the embodiment of this disclosure are in use.
FIG. 12 is a schematic view for illustrating a cross-sectional configuration of the imaging apparatus (200) according to the embodiment of this disclosure.
FIG. 13 is a schematic view for illustrating a cross section of a subject person (7) in the embodiment of this disclosure.

### Description of Embodiments

Various embodiments of this disclosure are described with reference to the accompanying drawings. The same components in the drawings are denoted by the same reference symbols.

### <First Embodiment>

### 1. Overview of Imaging System 1

An imaging system 1 according to this disclosure is mainly used to image an intraoral image of a test subject person so as to obtain an object image. In particular, the imaging system 1 is used to image an image of an intraoral region around the back of a throat, more specifically, a pharynx. Thus, the imaging system 1 according to this disclosure, which is used to image an image of the pharynx, is mainly described below. However, the pharynx is an example of a region being subjected to imaging. It is apparent that the imaging system 1 according to this disclosure can be suitably used for any other intraoral regions.

As an example, the imaging system 1 according to this disclosure is used to image an image of a pharynx in an oral cavity of a subject person to determine a probability of influenza infection of the subject person. Thus, now, there is described a case in which the imaging system 1 is used to determine a probability of influenza infection. However, the determination of a probability of influenza infection is a mere example of the use of the imaging system of this disclosure. It is apparent that the imaging system of this disclosure may be suitably used for the determination of any diseases that may appear as different intraoral findings as a result of infection. Examples of such diseases include streptococcal infections, adenovirus infections, Epstein-Barr (EB) virus infections, mycoplasma infections, and hypertensions.

In this disclosure, the terms relating to diseases, such as "determination" and "diagnosis", are used. However, these terms do not always refer to a definitive determination or diagnosis made by a doctor. For example, it is apparent that the terms "determination" and "diagnosis" may also refer to a determination and a diagnosis made by a processing device 100 included in the imaging system 1 when an imaging subject person himself or herself or a user other than doctors uses the imaging system 1 of this disclosure.

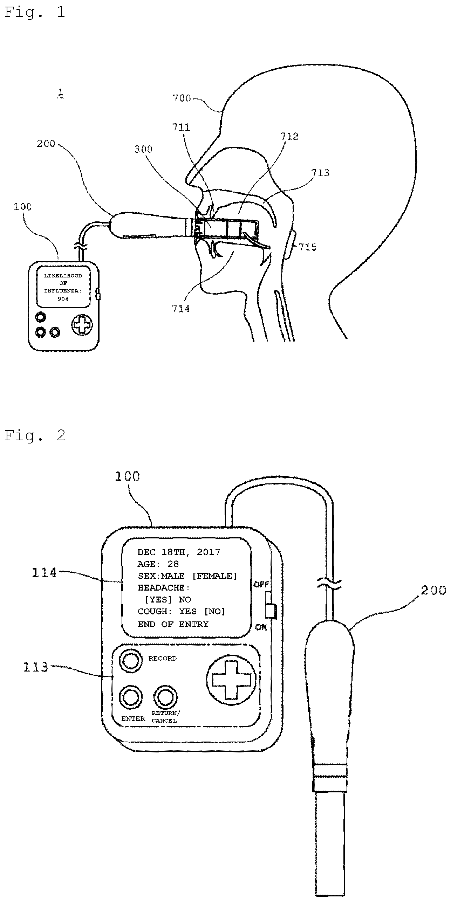

FIG. 1 is a view for illustrating a state in which the imaging system 1 according to an embodiment of this disclosure is in use. As illustrated in FIG. 1, the imaging system 1 according to this disclosure includes the processing device 100 and an imaging apparatus 200. A user inserts an auxiliary member 300 into an oral cavity of a subject person, and inserts the imaging apparatus 200 into the auxiliary member 300 so that the imaging apparatus 200 is covered with the inserted auxiliary member 300. Thus, the imaging apparatus 200 is used to image an intraoral image. More specifically, the auxiliary member 300 is first inserted into an oral cavity by a user (may be a subject person 700 himself or herself or may be different from the subject person 700) of the imaging apparatus 200 of the imaging system 1. At this time, a distal end of the auxiliary member 300 is inserted to pass between incisor teeth 711 to reach the vicinity of a soft palate 713. Then, a distal end of the imaging apparatus 200 is inserted into the auxiliary member 300 under a state in which the auxiliary member 300 is inserted into the oral cavity. Thus, the imaging apparatus 200 is inserted to pass between the incisor teeth 711 to reach the vicinity of the soft palate 713. At this time, a tongue 714 is pushed downward by the auxiliary member 300 (functioning as a tongue depressor), to thereby restrict movement of the tongue 714. At the same time, the soft palate 713 is pushed upward by the distal end of the auxiliary member 300. As a result, a clear field of view is ensured for the imaging apparatus 200 so that an image of a pharynx 715 located in front of the imaging apparatus 200 is imaged.

The thus imaged object image (image of the pharynx 715) is transmitted from the imaging apparatus 200 to the processing device 100 that is connected to the imaging apparatus 200 in a wired manner so as to be communicable therewith. After a processor of the processing device 100 that has received the object image processes programs stored in a memory of the processing device 100, a probability of influenza infection is determined based on the object image. Then, a result thereof is output to, for example, a display.

### 2. Configuration of Imaging System 1

FIG. 2 is a schematic view of the imaging system 1 according to the embodiment of this disclosure. As illustrated in FIG. 2, the imaging system 1 includes the processing device 100 and the imaging apparatus 200. The imaging apparatus 200 is connected to the processing device 100 in a wired manner so as to be communicable therewith. The processing device 100 receives an operation input by the user to control imaging performed by the imaging apparatus 200. Further, the processing device 100 processes the object image imaged by the imaging apparatus 200 to determine a probability of influenza infection of the subject person. Further, the processing device 100 outputs a result of determination to notify, for example, the user or the subject person of the result of determination.

At least the distal end of the imaging apparatus 200 is inserted into an oral cavity of the subject person to image an intraoral image, in particular, an image of a pharynx. Specific imaging processing is described later. The imaged object image is transmitted to the processing device 100 via a wired connection cable.

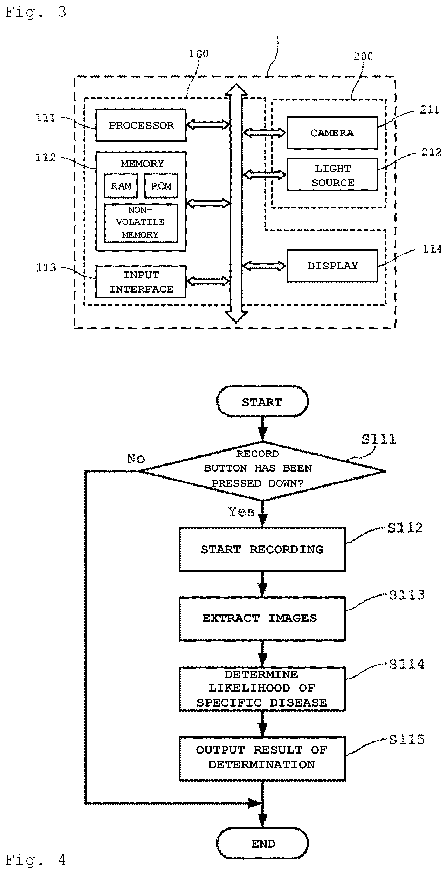

FIG. 3 is a block diagram for illustrating a configuration of the imaging system 1 according to the embodiment of this disclosure. As illustrated in FIG. 3, the imaging system 1 includes the processing device 100 and the imaging apparatus 200. The processing device 100 includes a processor 111, a memory 112, an input interface 113, and a display 114. The imaging apparatus 200 includes a camera 211 and a light source 212. The above-mentioned components are electrically connected to each other through control lines and data lines. The imaging system 1 is not required to include all the components illustrated in FIG. 3. One or a plurality of the components may be omitted, or other components may be additionally provided. For example, the imaging system 1 may include, for example, a battery for driving the components or a communication interface for transmitting the result of processing performed by the imaging system 1 to an outside and receiving an instruction and a command input from the outside.

The processor 111 functions as a control unit that controls other components of the imaging system 1 based on the programs stored in the memory 112. The processor 111 controls driving of the camera 211 and driving of the light source 212, and stores the object image received from the imaging apparatus 200 to process the stored object image based on the programs stored in the memory 112. More specifically, the processor 111 receives an instruction input to the input interface 113 by the user to execute processing based on the programs stored in the memory 112. The processing includes, for example, processing for turning on the light source 212 to instruct the camera 211 to perform imaging, processing for extracting a still image to be used for a determination from the imaged images, processing for performing a predetermined process on the extracted image to determine likelihood of a predetermined disease, and processing for outputting a result of determination to, for example, the display 114. The processor 111 mainly includes one or a plurality of CPUs. However, the processor 111 may suitably include a GPU or other units in combination therewith.

The memory 112 is formed of, for example, a RAM, a ROM, a non-volatile memory, or an HDD, and functions as a storage unit. The memory 112 stores instructions and commands for various kinds of control on the imaging system 1 according to this embodiment as programs. More specifically, the memory 112 receives an instruction input to the input interface 113 by the user to store the programs that allow the processor 111 to execute the processing. The processing includes, for example, the processing for turning on the light source 212 to instruct the camera 211 to perform imaging, the processing for extracting a still image to be used for determination from the imaged images, processing for performing a predetermined process on the extracted image to determine likelihood of a predetermined disease, and the processing for outputting a result of determination to, for example, the display 114. Further, the memory 112 stores, in addition to the programs, the object image imaged by the camera 211 of the imaging apparatus 200, a result of determination of likelihood of a disease, which is made by the processor 111, various kinds of information about subject persons including the subject person 700, and other information.

The input interface 113 functions as an input unit that receives an instruction issued to the processing device 100 and the imaging apparatus 200, which is input by the user. Examples of the input interface 113 include, as illustrated in FIG. 2, a "record button" for instructing the imaging apparatus 200 to start or end recording, an "enter button" for making various selections, a "return/cancel button" for returning to the previous page or image or canceling the input enter operation, a directional pad for moving, for example, an icon on the display 114, and an ON/OFF key for powering on and off the processing device 100. Although not particularly shown, a touch panel may be used as the input interface 113. The touch panel is provided so as to overlap the display 114, and has an input coordinate system corresponding to a display coordinate system of the display 114. As a method of detecting an instruction input by the user with use of the touch panel, any method such as a capacitive touch method or a resistive touch method may be used.

The display 114 functions as a display unit for displaying the object image imaged by the imaging apparatus 200 and outputting a result of determination made by the processor 111. The display 114 is formed of a liquid crystal panel. However, the display 114 is not limited to a liquid crystal panel, and may also be formed of, for example, an organic electroluminescence (EL) display or a plasma display.

The camera 211 functions as an imaging unit. The imaging unit is driven in response to an instruction output from the processing device 100, and detects light reflected by the oral cavity being an object, to thereby generate the object image. To detect the light, the camera 211 includes, as an example, a CMOS image sensor, a lens system, and a drive system. The lens system and the drive system are provided to achieve desired functions. The image sensor is not limited to a CMOS image sensor, and other sensors such as a CCD image sensor may be used. Although not particularly shown, the camera 211 may have an autofocus function. It is preferred that the camera 211 be set so as to be focused on a specific region in front of a lens, for example. Further, the camera 211 may have a zooming function. It is preferred that the camera 211 be set to image an image at a suitable magnification in accordance with a size of a pharynx or influenza follicles.

In this embodiment, the camera 211 is inserted into the oral cavity of the subject person, and is used to image an image of the pharynx located in the back of the oral cavity. Thus, a distance between the camera 211 and the object is relatively small. Thus, the camera 211 has an angle of view (2θ) that allows values calculated by [(distance from distal end portion of camera 211 to rear wall of pharynx)*tanθ] to be 20 mm or larger as a vertical range and 40 mm or larger as a horizontal range. The use of the camera having the above-mentioned angle of view enables imaging over a wider range even when the camera 211 and an object are close to each other. Thus, a general camera may be used as the camera 211, or a so-called wide angle camera or super-wide angle camera may be used.

Further, in this embodiment, influenza follicles, which are a target of imaging by the camera 211, are formed at the pharynx in the oral cavity. The pharynx is generally located at the back in a depth direction. Thus, when a depth of field is shallow, a region between an anterior part of the pharynx and a posterior part of the pharynx may be out of focus. Hence, it is difficult to obtain an appropriate object image to be used for the determination made by the processing device 100. Thus, the camera 211 has a depth of field of at least 20 mm or larger, preferably, 30 mm or larger. The use of the camera having such a depth of field allows an in-focus object image of any region between the anterior part of the pharynx and the posterior part of the pharynx to be obtained.

The light source 212 is driven in response to an instruction output from the processing device 100, and functions as an optical source unit for irradiating the oral cavity with light. The light source 212 includes one or a plurality of light sources. In this embodiment, the light source 212 is formed of one or a plurality of light-emitting diodes (LEDs). Light having a predetermined frequency band is radiated from each of the LEDs in a direction toward the oral cavity. As light emitted from the light source 212, light having a desired band selected from an ultraviolet light band, a visible light band, and an infrared light band, or a combination thereof is used. In particular, when influenza follicles are irradiated with light having a short-wavelength band in the ultraviolet light band, a specific component in the influenza follicles reacts therewith. Thus, likelihood of a disease can be more reliably determined.

In this embodiment, description has been given of the case in which the processing device 100 and the imaging apparatus 200 are connected to each other through a wired connection cable so as to be communicable with each other. However, it is apparent that the connection therebetween is not limited to the wired connection. The processing device 100 and the imaging apparatus 200 may be connected through wireless communication.

### 3. Flow of Processing Executed by Processing Device 100

FIG. 4 is a flowchart for illustrating processing executed by the processing device 100 in the embodiment of this disclosure. More specifically, FIG. 4 is a flowchart for illustrating processing executed by the processor 111 of the processing device 100 in predetermined cycles based on the programs stored in the memory 112.

In this embodiment, at least one image that satisfies predetermined conditions is extracted from object images (video image) imaged by the imaging apparatus 200. For example, the video image imaged by the imaging apparatus 200 includes still images at a predetermined film rate. The still images include those imaged under desirable imaging conditions and those imaged under undesirable imaging conditions. In this case, several frames (still images) imaged under desirable imaging conditions are selected from the imaged video image. For example, conditions under which a specific disease (for example, influenza) is diagnostically determined with high accuracy are found in advance based on a number of pieces of similar image data. The conditions include all or some of a specific imaging angle, a degree of light emission from a specific light source, wideness of a specific field of view, a position at which a specific region (for example, a pharynx) is extracted, and a degree of focus on the specific region. The conditions are set as criteria for determining whether imaging conditions are desirable or undesirable. Then, the likelihood of a disease is determined by processing several still images extracted based on the criteria. Machine learning such as a deep learning function may be used for extraction processing. In this embodiment, description has been given of the case in which still images are extracted from a video image. However, a plurality of still images may be imaged so that several appropriate ones are extracted therefrom. As another example, only the number of still images used for the determination may be imaged, and the extraction processing may be omitted.

As illustrated in FIG. 4, the processor 111 determines whether or not a record button of the input interface 113 has been pressed down (Step S111). When it is determined that the record button has not been pressed down, the processor 111 ends the flow of processing without performing subsequent steps. Meanwhile, when it is determined that the record button has been pressed down, the processor 111 drives the light source 212 of the imaging apparatus 200, and controls the imaging apparatus 200 so as to start picking up a video image with the camera 211 of the imaging apparatus 200 (Step S112). Meanwhile, the user mounts the auxiliary member 300 into an oral cavity 712 of a subject person, and inserts the imaging apparatus 200 into the mounted auxiliary member 300. Then, the processor 111 controls the imaging apparatus 200 to image an object video image of the pharynx.

Next, the processor 111 extracts still images appropriate for the determination from still images appropriate for the determination from a plurality of still images included in the acquired video image (Step S113). In this step, the extracted still images may be shown on the display 114. Then, the processor 111 determines likelihood of a specific disease (for example, likelihood of influenza) based on the extracted still images and a determination algorithm (Step S114). Then, the processor 111 performs control so as to output the result of determination to, for example, the display 114 (Step S115). As a result, a series of processing steps ends.

As an example, machine learning is used for the determination based on the determination algorithm in Step S114. More specifically, an image of the pharynx of an influenza patient and a label attached thereto, which indicates whether or not the patient is infected with influenza, are collected in, for example, a medical institution. At this time, a ground truth label is attached based on, for example, a result of an influenza rapid test, a PCR test, or a virus isolation culture test, each using a swab, which the patient has undergone. The PCR test and the virus isolation culture test require several weeks to give results. However, the PCR test and the virus isolation culture test have extremely high accuracy. Thus, the results thereof are suitable as ground truth labels.

Next, training data (image data) labelled with a result of determination of the PCR test or the virus isolation culture test as a ground truth is generated. Machine learning is performed based on the training data to generate the determination algorithm. The determination algorithm serves to determine, when an image is given, whether or not the image shows the likelihood of influenza. The determination algorithm enables a probability of influenza to be quantified, for example, as "98.5%". The probability may be indicated by an appropriate combination of, for example, a "positive" or "negative" determination and a grade evaluation as "high", "middle", or "low".

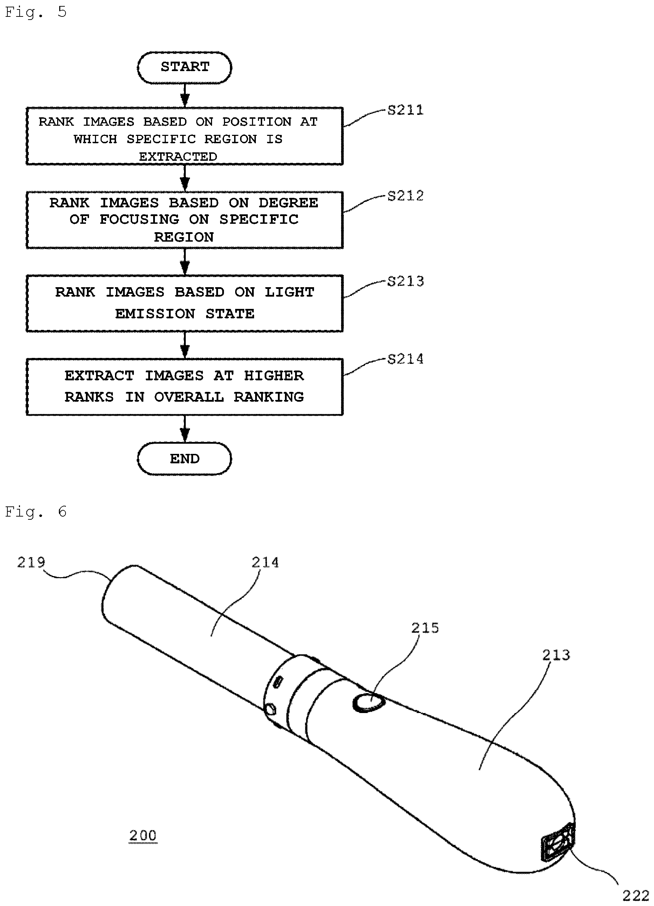

FIG. 5 is a flowchart for illustrating processing executed by the processing device 100 in the embodiment of this disclosure. More specifically, FIG. 5 is a flowchart for illustrating an example of specific extraction processing executed in Step S113 of FIG. 4 by the processor 111 of the processing device 100 based on the programs stored in the memory 112.

As illustrated in FIG. 5, the processor 111 ranks the images extracted from the video image imaged by the imaging apparatus 200 based on a position at which a target of imaging is extracted (Step S211). Next, the processor 111 ranks the images extracted from the video image based on the degree of focusing on a specific region (Step S212). Next, the processor 111 ranks the images extracted from the video image based on a light emission state from the light source (Step S213) . Then, the processor 111 selects several images of higher ranks in overall ranking (Step S214). The extraction processing is an example, and criteria such as wideness of a field of view and a shooting angle may be used.

### 4. Configuration of Imaging Apparatus 200

The imaging system 1 according to this disclosure includes the imaging apparatus 200 that picks up an image to be processed in the processing device 100. FIG. 6 is a perspective view for illustrating a configuration of the imaging apparatus 200 according to the embodiment of this disclosure. As illustrated in FIG. 6, the imaging apparatus 200 includes a main body 214, a handgrip 213, a diffuser plate 219, and an I/O interface 222. The main body 214 has an inner surface inside which the camera 211 is accommodated, and can guide light emitted from the light source 212 therein. The handgrip 213 is connected to a proximal end 220 of the main body 214, and is held by the user. The diffuser plate 219 is provided to a distal end of the main body 214. The I/O interface 222 is provided to an end portion of the handgrip 213. The wired connection cable has one end connected to the processing device 100 and another end connected to the I/O interface 222. Information is transmitted to and received from the processing device 100 via the I/O interface 222.

In this embodiment, the imaging apparatus 200 includes a record button 215. Thus, the user can instruct to start and end imaging with use of the record button of the input interface 113 of the processing device 100, but the user can also instruct to start and end imaging with use of the record button 215.

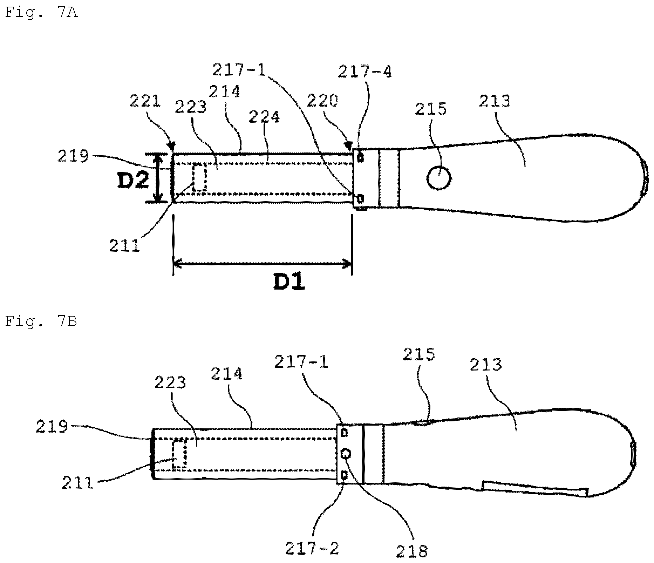

FIG. 7A is a schematic view for illustrating a top configuration of the imaging apparatus 200 according to the embodiment of this disclosure. Further, FIG. 7B is a schematic view for illustrating a side configuration of the imaging apparatus 200 according to the embodiment of this disclosure. Now, with reference to FIG. 7A and FIG. 7B, a specific configuration of the imaging apparatus 200 is described.

As illustrated in FIG. 7A and FIG. 7B, the main body 214 is formed of a columnar member. The main body 214 has the proximal end 220 and a distal end 221, and has a predetermined length in a direction substantially parallel to a direction in which light is emitted from the light source 212. At least the distal end 221 of the main body 214 is inserted into the oral cavity.

The main body 214 is formed in a hollow cylindrical columnar shape with a perfect circular cross section. A wall portion 224 thereof may be made of any material that allows light to be guided therein, and may be made of a thermoplastic resin as an example. As the thermoplastic resin, for example, polyolefin-based resins, such as chain polyolefin-based resins (such as polypropylene-based resins) and cyclic polyolefin-based resins (such as norbornene-based resins), cellulosic ester-based resins, such as tri-acetylcellulose and di-acetylcellulose, polyester-based resins, polycarbonate-based resins, (meta)-acrylic resins, polystyrene-based resins, or a mixture or a copolymer thereof are used. Specifically, the main body 214 functions as a light guiding member for guiding light emitted from the light source into the oral cavity or in a direction toward the diffuser plate.

The main body 214 is hollow, and hence has an accommodation space 223 defined by an inner surface of the wall portion 224. The camera 211 is accommodated in the accommodation space 223. The main body 214 is only required to be formed in a columnar shape having the accommodation space 223. Thus, the accommodation space 223 is not required to have a cylindrical shape with a perfect circular cross section, and may have an ellipsoidal or polygonal sectional shape. Further, the main body 214 is not always required to be hollow.

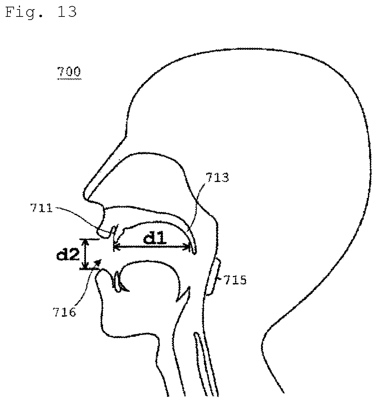

In this case, as an example, a length of the main body 214 is determined based on a positional relationship with the incisor teeth of the subject person. FIG. 13 is a schematic view for illustrating a cross section of the target 7 in the embodiment of this disclosure. More specifically, FIG. 13 is a view of the common subject person 7, for illustrating the oral cavity and the vicinity thereof in cross section. As illustrated in FIG. 13, the subject person 7 has an oral cavity extending from the incisor teeth 711 in a direction toward the back of a throat and the pharynx 715, which is an object, at the deepest end of the oral cavity. Thus, to image images of the pharynx 715, the imaging apparatus 200 is inserted so that its distal end reaches the vicinity of the soft palate 713. The subject person 7 has a distance d1 from the incisor teeth 711 to the soft palate 713. According to "Pharyngeal Cross-sectional Area in Sleep Apnea Syndrome" by Nobuo Ohya, J. Jpn. Bronchoesophagol. Soc., Vol. 40, No. 5, pp. 396 to 402, the distance d1 generally falls within a range of from about 100 mm to 200 mm.

Returning to FIG. 7A and FIG. 7B, the main body 214 has a distance D1 as a length from the distal end 221 to the proximal end 220 in this embodiment. It is preferred that this distance D1 be set to 100% or smaller of the distance d1 from the incisor teeth 711 to the soft palate 713, more preferably, to 80% or smaller. In general, when a foreign substance is inserted to reach the back of the throat, the subject person may feel nauseous. Meanwhile, when the main body 214 has a short length, the camera 211 may be too far from an object. The above-mentioned distance D1 can prevent nausea and maintain an appropriate distance from the object at the same time.

The handgrip 213 has a distal end connected to the proximal end 220 of the main body 214. The user holds the handgrip 213 to perform an operation such as removal and insertion of the imaging apparatus 200. The handgrip 213 has a shape suitable to be held in a user's palm. Specifically, the handgrip 213 is tapered relatively toward its distal end connected to the main body 214 and is upcurved toward its proximal end on the opposite side of the main body 214. In this embodiment, the handgrip 213 has a perfect circular cross section. However, the cross section of the handgrip 213 is not required to be perfect circular, and may also be ellipsoidal or polygonal.

In this case, as an example, a width (distance D2) of the main body 214 in a direction perpendicular to a direction that connects the distal end 221 and the proximal end 220 of the main body 214 is determined based on a relationship with an opening width of a subject person's mouth in a vertical direction. As illustrated in FIG. 13, the subject person 7 has a distance d2 as an opening width of a mouth in the vertical direction. According to "A Statistical Evaluation of Normal Maximal Mouth Opening of Japanese Adults" by Hiroyasu Tsukahara et al., Japanese Journal of Oral and Maxillofacial Surgery, Vol. 44, No. 2, pp. 159 to 167, an average of the distances d2 of ordinary adult males falls within a range of from 3.5 cm to 4.0 cm.

Returning to FIG. 7A and FIG. 7B, the imaging apparatus 200 of this embodiment is inserted together with the auxiliary member 300 into the width equal to the distance d2. The user is required to image images while observing the oral cavity through a gap defined by the imaging apparatus 200 and the auxiliary member 300, which are inserted into the oral cavity. Thus, it is beneficial not to impair visibility of the user under a state in which the imaging apparatus 200 is inserted. Thus, it is preferred that the distance D2 of the main body 214 be 80% or smaller of the distance d2 being the opening width of the mouth in the vertical direction, preferably, a width of 60% or smaller or 3.2 cm or smaller, more preferably, 2.4 cm or smaller.

The handgrip 213 has engagement protrusions 217 and a positioning protrusion 218 in the vicinity of the proximal end 220 of the main body 214. The engagement protrusions 217 and the positioning protrusion 218 are formed so as to enable positioning of the auxiliary member 300. The engagement protrusions 217 are engaged with engagement protrusions 318 of the auxiliary member 300. Further, the positioning protrusion 218 is inserted into an insertion hole 321 of the auxiliary member 300, to thereby position the imaging apparatus 200 and the auxiliary member 300 with respect to each other. In this embodiment, as the engagement protrusions 217 of the main body 214, a total of four engagement protrusions (engagement protrusions 217-1, 217-2, 217-3, and 217-4) are arranged at equal intervals in the vicinity of the proximal end 220 of the main body 214 on a surface of the handgrip 213. A single positioning protrusion 218 is arranged between the engagement protrusions 217 on the surface of the handgrip 213 in the vicinity of the proximal end 220 of the main body 214. However, the arrangement of the engagement protrusions 217 and the positioning protrusion 218 is not limited to that described above. Only any one of a set of the engagement protrusions 217 and the positioning protrusion 218 may be formed. Further, any number of engagement protrusions 217 and positioning protrusions 218 may be formed as long as one or a plurality of engagement protrusions 217 and one or a plurality of positioning protrusions 218 are formed.

The diffuser plate 219 is arranged at the distal end 221 of the main body 214, and diffuses light, which has been emitted from the light source 212 and has passed through the main body 214, into the oral cavity. The diffuser plate 219 has a shape in conformity with a sectional shape of a part of the main body 214, which is formed so as to be capable of guiding the light. In this embodiment, the main body 214 is formed in a hollow cylindrical shape. Thus, the diffuser plate 219 is also formed to have a donut-like sectional shape in conformity with the shape of the main body 214.

The camera 211 is used to detect the light, which has been diffused by the diffuser plate 219 to be radiated into the oral cavity and has been reflected by the object, to generate an object image. The camera 211 is arranged inside the inner surface of the wall portion 224 of the main body 214, specifically, in the accommodation space 223 defined inside the main body 214. In this embodiment, only one camera 211 is provided. However, the imaging apparatus 200 may include a plurality of cameras. When the object images are generated with use of a plurality of cameras, the object images include information about three-dimensional shapes of influenza follicles. As a result, the influenza follicles are more accurately detected based on the determination algorithm to enable determination of a probability of influenza infection. Further, in this embodiment, the camera 211 is arranged in the accommodation space 223 of the main body 214. However, the camera 211 may also be provided to the distal end 221 of the main body 214 or to the main body 214 (may be arranged in the main body 214 or on an outer periphery of the main body 214).

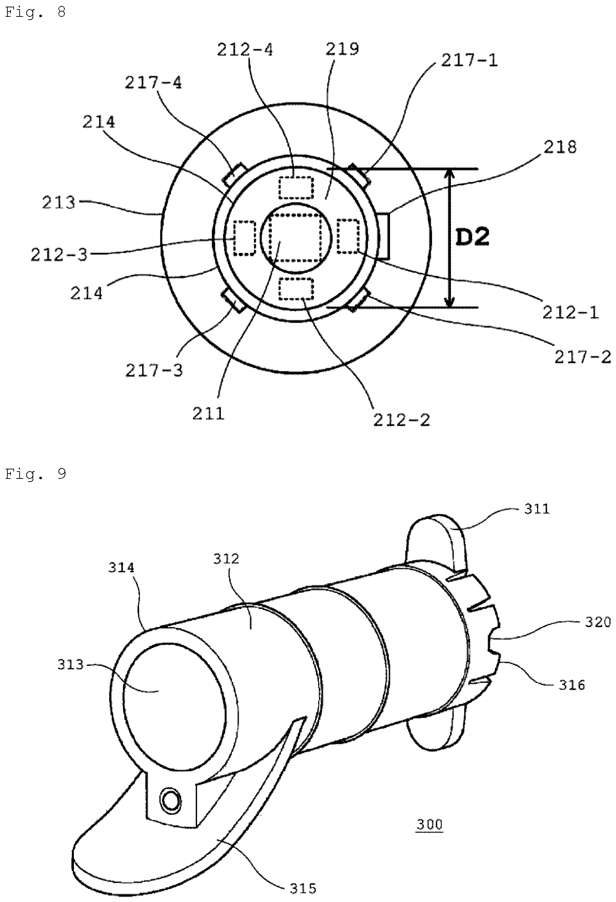

FIG. 8 is a schematic view for illustrating a front configuration of the imaging apparatus 200 according to the embodiment of this disclosure. More specifically, FIG. 8 is a view of the imaging apparatus 200 illustrated in FIG. 7A when viewed from the distal end 221 of the main body 214. As illustrated in FIG. 8, as described above, the imaging apparatus 200 includes the handgrip 213 having the distance D2 as the width. The main body 214 is arranged on a front side of the handgrip 213. Further, the camera 211 is provided on a rear side of the diffuser plate 219 having a donut-like shape so as to be located inside a hole of the donut-like shape in front view.

As described with reference to FIG. 7A and FIG. 7B, four engagement protrusions 217-1 to 217-4 and one positioning protrusion 218 are formed on the distal end side of the handgrip 213. Among the engagement protrusions 217-1 to 217-4 and the positioning protrusion 218, the engagement protrusions 217-1 to 2170-4 are arranged at equal intervals along an outer periphery of the distal end portion of the handgrip 213. Further, the positioning protrusion 218 is arranged between the engagement protrusions 217-1 and 217-2.

The light source 212 is provided to a rear part of the main body 214 behind the diffuser plate 217, specifically, at a predetermined position on a side closer to the handgrip 213. Light is emitted from the light source 212 in a direction toward the main body 214. The light, which has passed through the main body 214, is diffused into the oral cavity through the diffuser plate 219. In this embodiment, the light source 212 is arranged in conformity with the sectional shape of the main body 214. Specifically, four light sources (light sources 212-1, 212-2, 212-3, and 212-4) are arranged at equal intervals on a board 225 (not shown in FIG. 8) provided so as to be directed to the proximal end 220 of the main body 214.

### 3. Configuration of Auxiliary Member 300

FIG. 9 is a perspective view for schematically illustrating a configuration of the auxiliary member 300 in the embodiment of this disclosure. The auxiliary member 300 is an auxiliary tool to be used to image intraoral images of the subject person 7. Thus, the auxiliary member 300 is not always needed when intraoral images are imaged by using the imaging apparatus 200, and is not required to be used when the user or the subject person does not desire to use the auxiliary member 300. At least a distal-end part of the imaging apparatus 200 is inserted into the auxiliary member 300. Thus, it is preferred that the auxiliary member 300 have translucency in view of ensuring a clear field of view without becoming an obstacle to the imaging apparatus 200.

In this embodiment, the auxiliary member 300 is manufactured by integral molding of a resin. However, the auxiliary member 300 may be made of other materials such as paper, cloth, a metal, or a combination thereof. Further, it is desired that the auxiliary member 300 be of disposable type. However, the auxiliary member 300 may also be of reusable type.

As illustrated in FIG. 8, the auxiliary member 300 includes a main body 312, gripping plates 311, and a tongue depressor 315. The main body 312 is formed in a tubular shape. The gripping plates 311 are provided to a proximal end 316 of the main body 312. The tongue depressor 315 is arranged in the vicinity of a distal end 314 of the main body 312.

The main body 312 is formed so as to cover at least a part of the main body 214 of the imaging apparatus 200 on the distal end 221 side, and thus is formed in a cylindrical shape having a predetermined length corresponding to a length of the main body 214. The main body 312 has a proximal end-side opening 320 on the proximal end 316 side and a distal end-side opening 313 on the distal end 314 side. The proximal end-side opening 320 receives the imaging apparatus 200 inserted therein. The distal end-side opening 313 allows light emitted from the light source 212 of the imaging apparatus 200 and reflected light from the object to pass therethrough. In this embodiment, the distal end 314 of the auxiliary member 300 is first inserted into the oral cavity.

Further, in this embodiment, the main body 214 of the imaging apparatus 200 has an inner diameter and an outer diameter that are substantially constant from the proximal end 220 to the distal end 221. Thus, the main body 312 of the auxiliary member 300 is also formed to have substantially constant inner diameter and outer diameter in a longitudinal direction so as to conform with the shape of the main body 214. It is preferred that the main body 312 be formed to have a sectional shape in conformity with the sectional shape of the main body 214 of the imaging apparatus 200. However, the main body 312 may have any sectional shape, such as a perfect circular shape, an ellipsoidal shape, or a polygonal shape, as long as the main body 214 is insertable therein.

The gripping plates 311 are arranged along the distal end 316 of the main body 316. The gripping plates 311 are gripped by the user with a hand when the user inserts the auxiliary member 300 into the oral cavity of the subject person. In this embodiment, the gripping plates 311 also function as a regulating member. When the auxiliary member 300 is inserted into the oral cavity, distal end-side surfaces of the gripping plates 311 are brought into contact with, for example, lips of the subject person, to thereby regulate further insertion of the auxiliary member 300. In this embodiment, as the gripping plates 311, a pair of gripping plates are provided to the main body 312 on the proximal end 316 side so as to be symmetric in the vertical direction. However, a gripping plate may be formed in any shape such as a donut-like shape extending along the proximal end 316 of the main body 312.

The tongue depressor 315 is provided to a lower part (side closer to a tongue in the oral cavity) of the main body 312, and is formed in a blade-like shape extending in a direction toward the tongue. When images are imaged by the imaging apparatus 200, movement of the tongue of the subject person in front of the imaging apparatus 200 hinders the pickup of intraoral images. The tongue depressor 315 pushes the tongue downward to restrict the movement of the tongue in the oral cavity and prevent the tongue from being located in front of the imaging apparatus 200. Thus, in this embodiment, the tongue depressor 315 is formed in a blade-like shape. However, the tongue depressor 315 may have any shape as long as the above-mentioned function is achievable.

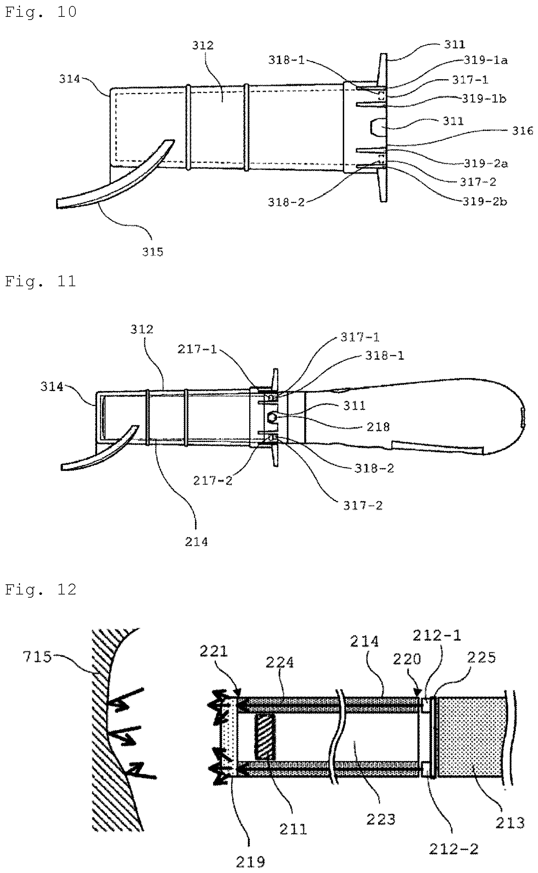

FIG. 10 is a side view for schematically illustrating a side configuration of the auxiliary member 300 in the embodiment of this disclosure. As illustrated in FIG. 10, as described above, the auxiliary member 300 includes the tongue depressor 315 in the vicinity of the distal end 314 of the main body 312 and the gripping plates 311 on the proximal end 316 side.

Further, the main body 312 has mechanisms for positioning the imaging apparatus 200 on the proximal end 316 side of the main body 312 when the imaging apparatus 200 is inserted from the proximal end 316 side. More specifically, the main body 312 includes a pair of grooves 319, regions 317, the engagement protrusions 318, and the insertion hole 321. The pair of grooves 319 are formed to extend in a direction perpendicular to an outer periphery of the main body 312 to have a predetermined length. The regions 317 are defined by the pairs of grooves 319. The engagement protrusions 318 are arranged in the regions 317, and are configured to be engaged with the engagement protrusions 217 formed on the distal end of the handgrip 213 of the imaging apparatus 200. The positioning protrusion 218 of the handgrip 213 is inserted into the insertion hole 321.

The engagement protrusions 318 are formed at positions corresponding to the engagement protrusions 217 formed on the handgrip 213 of the imaging apparatus 200 so as to protrude in a direction toward an inner surface of the main body 312. Thus, in this embodiment, four engagement protrusions 318-1, 318-2, 318-3, and 318-4 are arranged at positions corresponding to four engagement protrusions 217-1, 217-2, 217-3, and 217-4, respectively. Further, the insertion hole 321 is formed at a position corresponding to the positioning protrusion 218 formed on the handgrip 213 of the imaging apparatus 200. Thus, in this embodiment, one insertion hole 321 is formed at a position corresponding to the single positioning protrusion 218.

FIG. 11 is a schematic view for illustrating a state in which the imaging apparatus 200 and the auxiliary member 300 in the embodiment of this disclosure are in use. Now, the mechanisms provided to the proximal end 316 of the main body 312 in FIG. 10 are described with reference to FIG. 11. First, when the imaging apparatus 200 is inserted into the proximal end 316 of the auxiliary member 300 and reaches the vicinity of the distal end 314, the engagement protrusions 217 formed on the handgrip 213 of the imaging apparatus 200 so as to protrude outwardly therefrom and the engagement protrusions 318 formed in a direction toward the inner surface of the main body 312 of the auxiliary member 300 are brought into abutment with each other. In this embodiment, the engagement protrusions 318-1 to 318-4 of the main body 312 are arranged in the regions 317-1, 317-2, 317-3, and 317-4 defined by the pairs of grooves 319-1a and 319-1b, 319-2a and 319-2b, 319-3a and 319-3b, and 319-4a and 319-4b, respectively. Thus, when a force is further applied in a direction of insertion of the imaging apparatus 200 under a state in which the engagement protrusions 217 of the imaging apparatus 200 and the engagement protrusions 318 of the auxiliary member 300 are in abutment with each other, the regions 317, each being defined by one pair of grooves 319, are lifted up. As a result, the imaging apparatus 200 is more deeply inserted after the engagement protrusions 217 of the imaging apparatus 200 climb over the engagement protrusions 318 of the auxiliary member 300.

Next, when the imaging apparatus 200 is inserted to climb over the engagement protrusions 318 of the auxiliary member 300, the positioning protrusion 218 formed on the handgrip 213 of the imaging apparatus 200 so as to protrude outwardly therefrom is inserted into the insertion hole 321 of the auxiliary member 300. Then, the positioning protrusion 218 of the imaging apparatus 200 is brought into abutment against a wall surface of the insertion hole 321 formed on the distal end 314 side. As a result, further insertion of the imaging apparatus 200 in a direction toward the distal end 314 of the auxiliary member 300 is regulated.

Specifically, in this embodiment, the movement of the imaging apparatus 200 in the direction of insertion of the imaging apparatus 200 is regulated by the positioning protrusion 218 and the insertion hole 321 under a state in which the imaging apparatus 200 is fully inserted into the auxiliary member 300. The movement of the imaging apparatus 200 in a direction opposite to the above-mentioned direction, specifically, a direction of removal of the imaging apparatus 200 is regulated by the engagement protrusions 217 and the engagement protrusions 318.

### 6. Configuration of Light Source 212

FIG. 12 is a schematic view for illustrating a cross-sectional configuration of the imaging apparatus 200 according to the embodiment of this disclosure. As illustrated in FIG. 12, the light source 212 includes a total of four light sources 212-1, 212-2, 212-3, and 212-4 arranged on the board 225 provided on a side closer to the distal end of the handgrip 213. As an example, the light source 212 is formed of LEDs. Light having a predetermined frequency band is emitted from each of the LEDs in a direction toward the oral cavity. More specifically, the light emitted from the light source 212 is incident on the proximal end 220 of the main body 214, and is guided in a direction toward the diffuser plate 219 by the wall portion 224 of the main body 214. The light, which has reached the diffuser plate 219, is diffused inside the oral cavity by the diffuser plate 219. Then, the light diffused by the diffuser plate 219 is reflected by, for example, the pharynx 715, which is an object. When the reflected light reaches the camera 211, an object image is generated.

The light sources 212-1 to 212-4 may be configured so as to be independently controllable. For example, when light is emitted from some of the light sources 212-1 to 212-4, the object image can include a shadow of influenza follicles each having a three-dimensional shape. As a result, the object image includes information about the three-dimensional shape of the influenza follicles. Thus, influenza follicles can be more accurately detected based on the determination algorithm to enable determination of a probability of influenza infection.

Further, in this embodiment, the light sources 212-1 to 212-4 are provided on the proximal end 220 side of the main body 214. However, the light sources 212-1 to 212-4 may be provided to the distal end 221 of the main body 214 or to the main body 214 (inside the main body 214 or on the outer periphery of the main body 214).

In this embodiment, the diffuser plate 219 is used to prevent only a part of the oral cavity from being irradiated with the light emitted from the light source 212 and generate uniform light. Thus, as an example, micro lens arrays are formed on a surface of the diffuser plate 219 so that a lens-shaped diffuser plate having a suitable diffusion angle is used. In place of such a diffuser plate, a diffuser plate capable of diffusing light by other methods, such as a diffuser plate having a surface with randomly arranged micro concavities and convexities to achieve a light diffusing function, may be used. Further, the diffuser plate 219 may be formed integrally with the main body 214. Such a diffuser plate can be achieved by, for example, a method of forming micro concavities and convexities at the distal end portion of the main body 214.

Further, the diffuser plate 219 is provided on the distal end 221 side of the main body 214 in this embodiment. However, a position of the diffuser plate 219 is not limited to that described above. The diffuser plate 219 may be provided at any position between the light source 212 and the oral cavity being a target of irradiation. For example, the diffuser plate 219 may be provided to the distal end 221 of the main body 214 or to the main body 214 (inside of the main body 214 or on the outer periphery of the main body 214).

As described above, the imaging apparatus 200 according to this embodiment can be inserted into the oral cavity of the subject person to image images. Thus, in this embodiment, the likelihood of a disease can be appropriately determined by processing the object images obtained by the imaging.

### <Other Embodiments>

In the embodiment described above, description has been given of the case in which the processing device 100 and the imaging apparatus 200 are connected through a wired connection cable so as to be communicable with each other. However, the connection is not limited to the wired connection. The processing device 100 and the imaging apparatus 200 may be connected through wireless communication. In this case, for example, the object image imaged by the imaging apparatus 200 is transmitted to a remotely installed server device. When the server device is controlled to function as a processing device, the likelihood of a disease can be determined. As another example, the object image is transmitted to a remotely installed terminal device for a doctor. When the terminal device is controlled to function as a processing device, the determination of a disease can be accompanied by doctor's findings.

Further, in the embodiment described above, description has been given of the case in which the processing device 100 and the imaging apparatus 200 are connected through a wired connection cable so as to be communicable with each other. However, the configurations of the processing device 100 and the imaging apparatus 200 are not limited to those described above. The imaging apparatus 200 itself may have a determination function of the processing device 100 so as to determine the likelihood of a disease. In this case, a result of the determination may be, for example, output from an output unit of the imaging apparatus 200 or output to a terminal device of the user, which is connected to the imaging apparatus 200 in a wired or wireless manner.

Further, in the embodiment described above, description has been given of the case in which the imaging apparatus 200 and the auxiliary member 300 are formed separately. However, the configurations of the imaging apparatus 200 and the auxiliary member 300 are not limited to those described above, and the imaging apparatus 200 and the auxiliary member 300 may be formed integrally. For example, the imaging apparatus 200 may include a tongue depressor for restricting the movement of the tongue inside the oral cavity. The tongue depressor is provided to a surface of the main body 214, which is closer to the tongue, so as to be arranged on a side closer to the tongue in the oral cavity. In this manner, images can be imaged without using the auxiliary member 300.

### Reference Signs List

1 imaging system, 100 processing device, 200 imaging apparatus, 211 camera, 212, 212-1 to 212-4 light source, 213 handgrip, 214 main body, 215 record button, 217, 217-1 to 217-4 engagement protrusion, 218 positioning protrusion, 219 diffuser plate, 220 proximal end, 221 distal end, 222 I/O interface, 223 accommodation space, 224 wall portion, 225 board, 300 auxiliary member, 311 gripping plate, 312 main body, 313 distal end-side opening, 314 distal end, 315 tongue depressor, 316 proximal end, 317, 317-1 to 317-4 region, 318, 318-1 to 318-4 engagement protrusion, 319, 319-1a and 319-1b to 319-4a and 319-4b pair of grooves, 320 proximal end-side opening, 321 insertion hole

## Claims

1. An imaging apparatus, comprising:
a main body which has a proximal end and a distal end, and is formed into a columnar shape having a predetermined length between the proximal end and the distal end so as to enable insertion of at least the distal end into an oral cavity;
one or a plurality of light sources which are provided on the proximal end side or the distal end side of the main body, or to the main body, and are configured to emit light having a predetermined frequency band;
a diffuser plate which is provided on the proximal end side or the distal end side of the main body, or to the main body, and is configured to diffuse the light emitted from the light sources toward the oral cavity; and
a camera which is provided on the proximal end side or the distal end side of the main body, or to the main body, and is configured to image an object image based on reflected light which has been diffused by the diffuser plate and reflected from the oral cavity.

2. The imaging apparatus according to claim 1, wherein the object image comprises an image obtained by picking up an image of a pharynx.

3. The imaging apparatus according to claim 1 or 2, wherein the predetermined frequency band comprises an ultraviolet light band.

4. The imaging apparatus according to any one of claims 1 to 3, wherein the camera has a depth of field of at least 20 mm.

5. The imaging apparatus according to any one of claims 1 to 4, wherein the predetermined length is equal to or smaller than a distance from incisor teeth to a soft palate of a subject person.

6. The imaging apparatus according to any one of claims 1 to 5, wherein a width of the main body in a direction perpendicular to a direction that connects the proximal end and the distal end of the main body is 80% or smaller of an opening width of a subject person in a vertical direction.

7. The imaging apparatus according to any one of claims 1 to 6, further comprising a handgrip which is provided on the proximal end side of the main body, and is configured to be held by a user of the imaging apparatus.

8. The imaging apparatus according to any one of claims 1 to 7, wherein, when at least a part of the imaging apparatus is inserted into an auxiliary member having a tubular shape in a direction that connects the proximal end and the distal end of the main body, the imaging apparatus is covered with the auxiliary member.

9. The imaging apparatus according to claim 8, further comprising at least one engagement protrusion configured to be engaged with an engagement protrusion of the auxiliary member.

10. The imaging apparatus according to claim 8 or 9, further comprising a positioning protrusion which is to be inserted into an insertion hole of the auxiliary member, and is configured to enable positioning of the imaging apparatus and the auxiliary member.

11. The imaging apparatus according to any one of claims 1 to 10, wherein the main body functions as a light guiding member so as to guide light emitted from the one or the plurality of light sources to the diffuser plate.

12. An imaging apparatus, comprising:
a main body which has a proximal end and a distal end, and is formed into a columnar shape having a predetermined length between the proximal end and the distal end so as to enable insertion of at least the distal end into an oral cavity;
one or a plurality of light sources which are provided on the proximal end side or the distal end side of the main body, or to the main body, and are configured to emit light having a predetermined frequency band toward the oral cavity;
a camera which is provided on the proximal end side or the distal end side of the main body, or to the main body, and is configured to image an object image based on reflected light which has been emitted from the one or the plurality of light sources and reflected from the oral cavity; and
a tongue depressor which is provided to the main body, and is configured to restrict movement of a tongue inside the oral cavity.

13. An imaging system, comprising:
the imaging apparatus of any one of claims 1 to 12; and
a processing device which is connected to the imaging apparatus in a wired or wireless manner so as to be communicable with the imaging apparatus, and is configured to process an object image imaged by the imaging apparatus.
